(19) Europäisches Patentamt
European Patent Office
Office européen des brevets

(11) Publication number : 0 422 846 B1

# (12) EUROPEAN PATENT SPECIFICATION

(45) Date of publication of patent specification : 16.02.94 Bulletin 94/07

(51) Int. Cl.$^5$ : C07D 451/04, A61K 31/46

(21) Application number : 90310933.8

(22) Date of filing : 05.10.90

## (54) Aroyl-ureas.

(30) Priority : 07.10.89 GB 8922622

(43) Date of publication of application : 17.04.91 Bulletin 91/16

(45) Publication of the grant of the patent : 16.02.94 Bulletin 94/07

(84) Designated Contracting States : AT BE CH DE DK ES FR GR IT LI LU NL SE

(56) References cited : EP-A- 0 323 077

(73) Proprietor : JOHN WYETH & BROTHER LIMITED
Huntercombe Lane South Taplow
Maidenhead Berkshire, SL6 0PH (GB)

(72) Inventor : Ward, Terence James
43 Loosen Drive
Maidenhead, Berkshire (GB)
Inventor : White, Janet Christine
22 Starling Close
Wokingham, Berkshire (GB)
Inventor : Bradley, Gerald
49 Byfleet Road
New Haw, Weybridge, Surrey (GB)

(74) Representative : Brown, Keith John Symons et al
c/o Wyeth Laboratories Huntercombe Lane South
Taplow Maidenhead Berkshire SL6 0PH. (GB)

EP 0 422 846 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to novel aroyl urea derivatives, to processes for their preparation, to their use and to pharmaceutical compositions containing them.

GB 2213816A discloses a class of urea and carbamic acid derivatives, and the corresponding thio analogues of the general formula

A-X-NHCW-Y-B (I).

In this formula, A represents an aromatic radical of the formula

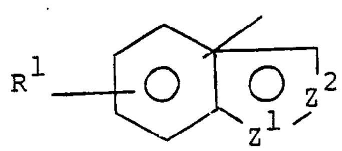

(a) ,

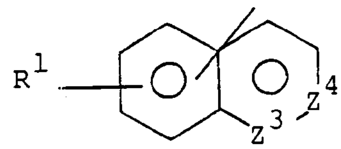

(b) ,

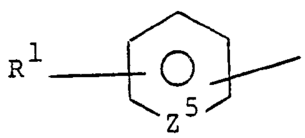

(c) or

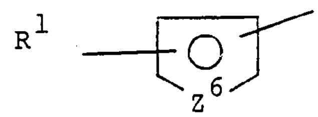

(d)

[where the free valence is attached to either fused ring of formula (a) or (b)]

$R^1$ represents hydrogen or one or more same or different substituents selected from lower alkyl, lower alkoxy, hydroxy, halogen, halo(lower)alkyl, amino, nitro, carboxamido, phenyl(lower)alkyloxy (in which the phenyl group may be optionally substituted by one or more lower alkyl, loweralkyloxy or halo substituents), (lower)alkylamino, di(lower)alkylamino or acylamino.

$Z^1$-$Z^2$ represents $CH_2$-CH, $NR^2$-CH, O-CH, S-CH, $CH_2$-N, O-N, S-N, $NR^2$-N, CH-$NR^2$ or N-$NR^2$, [where $R^2$ is hydrogen, (lower)alkyl or phenyl or phenyl(lower)alkyl in which the phenyl groups may optionally be substituted by one or more lower alkyl, lower alkyloxy or halo substituents]

$Z^3$-$Z^4$ represents CH=CH, O-$CH_2$ or N=CH

$Z^5$ represents N or CH

$Z^6$ represents O, S or NH

X represents a direct bond or CO,

W represents oxygen or sulphur,

Y represents NH or O,

B represents a saturated azacyclic ring of the formula

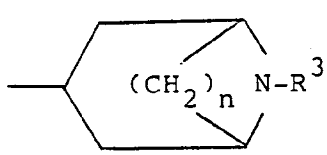

(II)

where n is 2,3 or 4 and $R^3$ is hydrogen, or (lower)alkyl,

or

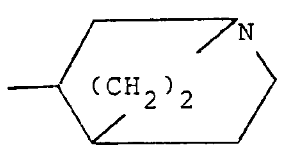

(III)

or the N-oxide thereof

or

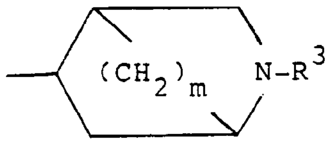

(IV)

where m is 1, 2 or 3 and $R^3$ has the meaning given above
or

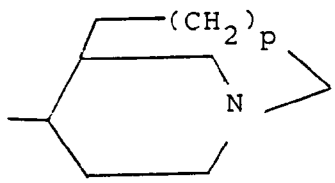

(V)

where p is 0, 1 or 2
or

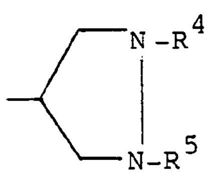

(VI)

where $R^4$ and $R^5$ are each hydrogen or lower alkyl
with the proviso that when X is a direct bond, A represents a group of formula (c) or (d) and W represents oxygen, then the ring (c) or (d) does not contain a substituent ortho to the -X-NHCW-Y-B side chain.

The compounds disclosed in GB 2213816A are stated to possess pharmacological activity. In particular they are stated to antagonise 5-$HT_3$ receptors in warm blooded animals and hence be of value in conditions where antagonism of 5-$HT_3$ receptors is desirable. A particular use of 5-$HT_3$-antagonists is as anxiolytics.

We have now found that a specific compound that falls within the general formula given above, but is not specifically disclosed in GB 2213816A, and its pharmaceutically acceptable acid salts, possess extremely potent 5-$HT_3$ antagonist activity when tested by standard pharmacological test procedures and is also very potent in the test for potential anxiolytic activity. Accordingly the present invention provides the novel compounds, (endo)-N-[[(8-methyl-8-azabicyclo[3.2.1]octan-3-yl)amino]carbonyl]-2-(cyclopropylmethoxy)benzamide and its pharmaceutically acceptable acid addition salts. The free base has the formula:

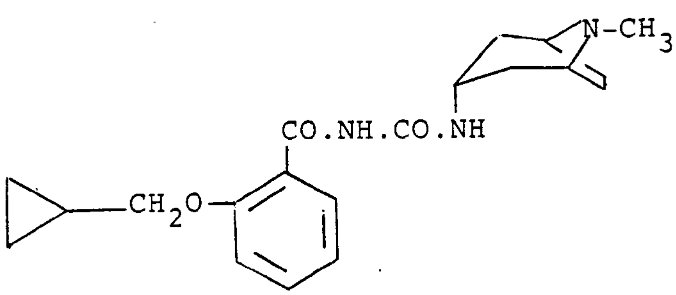

(VII)

We have found that the compounds of the present invention are surprisingly more potent as 5-$HT_3$-antagonists and specifically as potential anxiolytics in pharmacological test procedures than the other members of the class of compounds of formula (I) disclosed in GB 2213816A.

The compounds of the invention and the compounds disclosed in GB 2213816A are tested for 5-$HT_3$ antagonistic activity in the rat vagus by the following procedure:

The method is similar to that described by Ireland and Tyers, Br. J. Pharmac., 1987, 90, 229-238 and is

dependent upon the ability of 5-HT to depolarize the vagus nerve in vitro.

Segments of the vagus nerve from Sprague-Dawley rats were placed in a perspex chamber and perfused with Krebs solution. Electrodes, positioned at either end of the nerve segment, were used to record the potential differences which ensued upon the addition of various concentrations of 5-HT to one end of the nerve segment.

Concentration-response curves to 5-HT were obtained in this manner prior to and following equilibration of the nerve segment with Krebs solution containing the test-substance. A Schild analysis was performed on these results in order to obtain a measure of antagonist potency, expressed as a $pA_2$ value.

Results are given in the table below:-

TABLE A

| Compound | $pA_2$ |
|---|---|
| Compound of invention | 8.8 |
| Compounds of GB 2213816A | |
| Example 1 | 8.5 |
| 2 | <6.5 |
| 5 | 8.0 |
| 7 | 7.9 |
| 12 | 8.5 |
| 13 | 7.4 |
| 14 | <6.5 |
| 15 | <6.5 |
| 16 | <6.5 |
| 17 | <6.5 |
| 18 | <6.5 |
| 19 | 6.9 |
| 20 | <6.5 |
| 21 | 8.35 |
| 22 | 8.7 |
| 23 | 7.8 |
| 24 | <6.5 |
| 25 | 8.6 |
| 26 | 6.6 |
| 27 | 8.1 |
| 28 | 7.25 |
| 29 | 8.15 |
| 30 | 7.6 |

The table shows that the compound of the invention is more potent than the other compounds, especially

since the results are expressed in a log scale.

The compound of the invention and a representative number of compounds disclosed in GB 2213816A which had high $pA_2$ values in the $5\text{-}HT_3$-antagonist test procedure given above were tested for potential anxiolytic activity by a test procedure measuring mouse exploratory activity in a two-compartment light/dark box based upon the procedure of B Costall et al, Neuropharmacology, 1987, 26, 195-200 and J N Crawley et al, Pharmac. Biochem. Behav., 1980, 13, 167-170. The test involves observing groups of mice placed in an open topped box, one third of which is painted black and illuminated under a dim red light and partitioned from the remainder of the box which is painted white and brightly illuminated. Access between the two sections is via an opening in the centre of the partition. The groups of mice are treated with vehicle or test compound and various behavioural parameters of the animals are measured including the number of exploratory rearings made by the mice in each section and the number of times the mice cross lines marked on the floor of each section. For each treatment group the mean numbers of line crossings and rears in each section of the box are calculated. Differences between drug-treated groups and vehicle-treated controls are compared using Student's unpaired t-test. Standard anxiolytic agents significantly increase locomotion and rearing in the light section. Test compounds are considered to be active if they induce a similar set of changes and, in particular, if they produce a significant ($p < 0.05$) increase in rearing activity in the light section of the box.

The minimum effective dose (MED) in mg/kg is given in Table B below:-

TABLE B

| Compound | MED, mg/kg | |
|---|---|---|
| | s.c. | p.o. |
| Compound of invention | 0.01 | 0.1 |
| Compounds of GB 2213816A | | |
| Example 1 | 0.1 | |
| 5 | 0.1 | |
| 13 | 1 | |
| 21 | 1 | |
| 22 | 1 | 10 |
| 25 | 1 | 10 |

The compound of the invention is clearly more potent than the other compounds in the table.

The compound of the invention may be prepared by reacting 2-cyclopropylmethoxybenzoylisocyanate with (endo)-8-methyl-8-azabicyclo[3.2.1]octan-3-amine [ie 3-aminotropane]. The reaction may be carried out at, for example, room temperature in an organic solvent. The 2-cyclopropylmethoxy starting material may be prepared by methods known in the art, eg by the route shown in the following reaction scheme:-

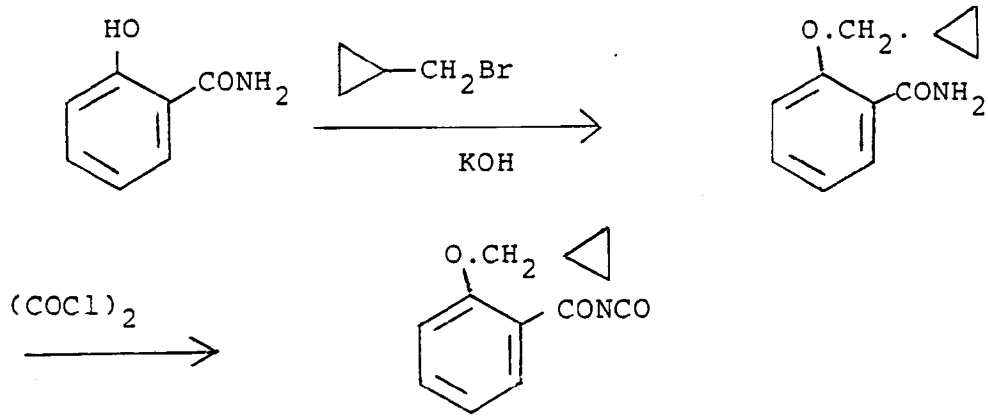


An alternative method of preparing the compound of the invention comprises reacting 2-cyclopropylmethoxybenzoylurea with (endo)-8-methyl-8-azabicyclo[3.2.1]octan-3-amine. Preferably the reaction is carried out by heating the reactants, eg at reflux temperature, in an inert organic solvent, for example toluene, pyridine, xylene, chlorobenzene, dimethylformamide or dioxan. The starting material may be prepared by methods known in the art, eg by the route shown in the following reaction scheme:-

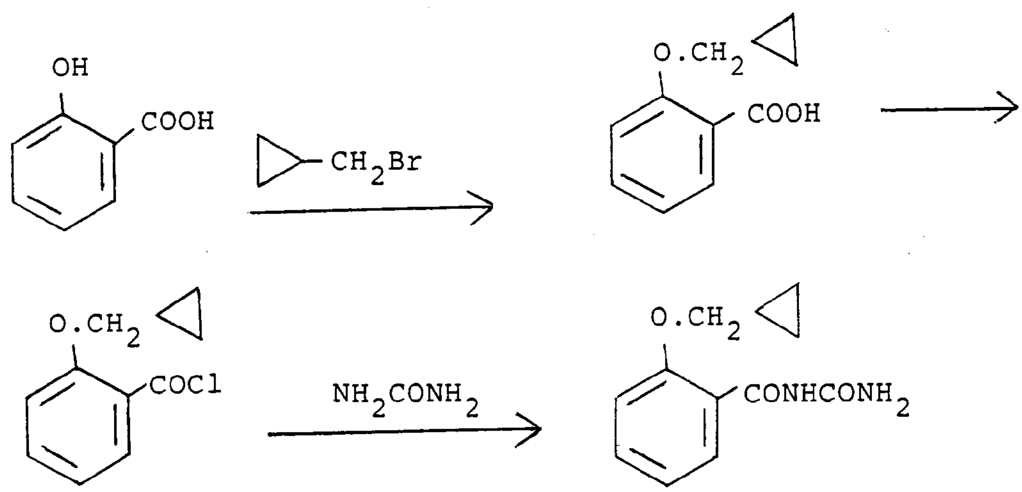


A further method of preparing the compound of the invention comprises reacting 2-(cyclopropylmethoxy)benzamide with (endo) -8-methyl-8-azabicyclo[3.2.1]octan-3-isocyanate. The isocyanate can be prepared by, for example, reacting (endo)-8-methyl-8-azabicyclo[3.2.1] octan-3-amine with phosgene. However it is preferable to prepare the isocyanate in situ by reacting the 2-(cyclopropylmethoxy)benzamide with (endo)-3-trichloroacetamido-8-methyl-8-azabicyclo[3.2.1]octane in presence of a base such as sodium hydroxide. The trichloroacetamide starting material may be prepared, for example, by reaction of (endo)-8-methyl-8-azabicyclo[3.2.1]octan-3-amine with trichloracetylchloride or hexachloracetone.

A further method of preparing the compound of the invention comprises acylating 1-[(endo)-8-methyl-8-azabicyclo[3.2.1]-octan-3-yl] urea with 2-(cyclopropylmethoxy)benzoic acid or an acylating derivative thereof, such as an acid halide (e.g. the acid chloride) or the anhydride.

Another method of preparing the compound of the invention comprises cyclopropylmethylating (endo)-N-[[(8-methyl-8-azabicyclo[3.2.1]octan-3-yl)amino]carbonyl]-2-hydroxybenzamide. The cyclopropylmethylation can be carried out with, for example, a cyclopropylmethyl halide e.g. cyclopropylmethyl bromide) in presence of a base such as KOH. The hydroxy substituted benzamide may be prepared in an analogous manner to the processes described above.

The compound of the invention may also be prepared by reacting cyclopropylmethanol with a compound of formula

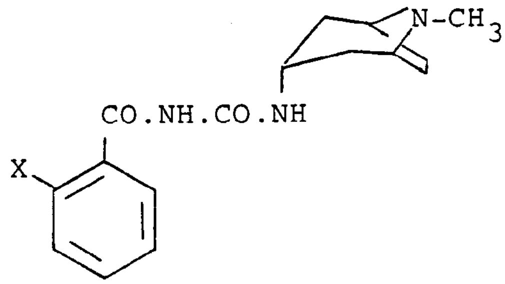

(VIII)

where X is a leaving group such as halogen (e.g. fluorine or chlorine) or an alkyl or arylsulphonyloxy group. The reaction may be carried out in the presence of a strong base in, for example, a dipolar aprotic solvent. The starting material of formula (VIII) may be prepared in an analogous manner to the processes described above.

A further method of preparing the compound of the invention comprises methylating a compound of formula

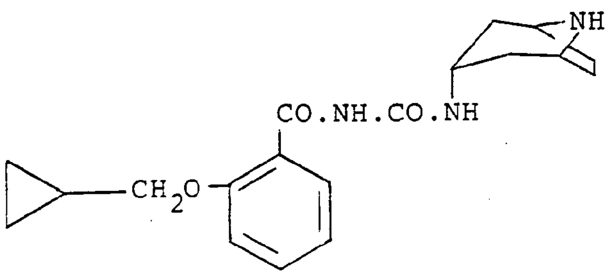

(IX)

The methylation may be carried out, for example, by reacting the compound of formula (IX) with a methyl halide. The starting material of formula (IX) may be prepared in an analogous manner to the processes described above; if necessary the amine group in the azabicyclo ring may be protected, e.g. by a benzyl group, when carrying out the processes and the protecting group removed subsequently.

If in the processes described above the compound of the invention is obtained as an acid addition salt, the free base can be obtained by basifying a solution of the acid addition salt. Conversely, if the product of the process is a free base, an acid addition salt, particularly a pharmaceutically acceptable acid addition salt may be obtained by dissolving the free base in a suitable organic solvent and treating the solution with an acid, in accordance with conventional procedures for preparing acid addition salts from base compounds.

Examples of acid addition salts are those formed from inorganic and organic acids, such as sulphuric, hydrochloric, hydrobromic, phosphoric, tartaric, fumaric, maleic, citric, acetic, formic, methane-sulphonic, p-toluenesulphonic, oxalic and succinic acids.

The invention further provides a compound of the invention for use in antagonising 5-$HT_3$ receptors in a mammal.

5-$HT_3$ antagonists may be useful in the treatment of neuro-psychiatric disorders such as anxiety, psychotic disorders (e.g. schizophrenia), dependancy on drugs or other substances of abuse, cognitive disorders; in the treatment of gastro-intestinal disorders such as emesis and nausea and in the treatment of migraine. Accordingly the invention provides the use of a compound of the invention for use in one or more of the above mentioned treatments. The invention also provides a method for one or more of the above mentioned treatments which comprises administering to a warm blooded animal in need thereof an effective amount of the compound of the invention.

For certain of the above mentioned conditions it is clear that the compounds may be used prophylactically as well as for the alleviation of acute symptoms. References herein to "treatment" or the like are to be understood to include such prophylactic treatment, as well as treatment of the acute conditions.

The compounds of the invention are particularly indicated for the treatment of anxiety.

In a further aspect the invention provides a pharmaceutical composition comprising a compound of the invention in association with a pharmaceutically acceptable carrier. Any suitable carrier known in the art can be used to prepare the pharmaceutical composition. In such a composition, the carrier is generally a solid or liquid or a mixture of a solid and a liquid.

Solid form compositions include powders, granules, tablets, capsules (e.g. hard and soft gelatin capsules),suppositories and pessaries. A solid carrier can be, for example, one or more substances which may also act as flavouring agents, lubricants, solubilisers, suspending agents, fillers, glidants, compression aids, binders or tablet-disintegrating agents; it can also be an encapsulating material. In powders the carrier is a finely divided solid which is in admixture with the finely divided active ingredient. In tablets the active ingredient is mixed with a carrier having the necessary compression properties in suitable proportions and compacted in the shape and size desired. The powders and tablets preferably contain up to 99%, e.g. from 0.03 to 99%, preferably 1 to 80% of the active ingredient. Suitable solid carriers include, for example, calcium phosphate, magnesium stearate, talc, sugars, lactose, dextrin, starch, gelatin, cellulose methyl cellulose, sodium carboxymethyl cellulose, polyvinylpyrrolidone, low melting waxes and ion exchange resins.

The term "composition" is intended to include the formulation of an active ingredient with encapsulating material as carrier to give a capsule in which the active ingredient (with or without other carriers) is surrounded by the carrier, which is thus in association with it. Similarly cachets are included.

Liquid form compositions include, for example, solutions, suspensions, emulsions, syrups, elixirs and pressurised compositions. The active ingredients, for example can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavouring agents, suspending agents, thickening agents, colours, viscosity regulators, stabilisers or osmo-regulators. Suitable examples of liquid carriers for oral and parenteral administration include water (particularly containing additives as above e.g. cellulose derivatives, preferably sodium carboxymethyl cellulose solution), alcohols (including monohydric alcohols and polyhydric alcohols e.g. glycerol and glycols) and their derivatives, and oils (e.g. fractionated coconut oil and arachis oil). For parenteral administration the carrier can also be an oily ester such as ethyl oleate and isopropyl myristate. Sterile liquid carriers are used in sterile liquid form compositions for parenteral administration.

Liquid pharmaceutical compositions which are sterile solutions or suspensions can be utilized by, for example, intramuscular, intraperitoneal or subcutaneous injection. Sterile solutions can also be administered intravenously. When the compound is orally active it can be administered orally either in liquid or solid composition form.

The compounds of the invention can also be administered by the nasal route. When formulated for nasal administration the compositions may comprise a compound of the invention in a liquid carrier; such compositions may be administered for example in the form of a spray or as drops. The liquid carrier may be water (which may contain further components to provide the desired isotonicity and viscosity of the composition). The composition may also contain additional excipients such as preservatives, surface active agents and the like. The compositions may be contained in a nasal applicator that enables the composition to be administered as drops or as a spray. For administration from an aerosol container the composition should also include a propellant.

Pharmaceutical compositions for treatment and/or prevention of nausea or vomiting may contain a cyclo-oxygenase inhibitor in addition to a compound of the invention. Examples of cyclo-oxygenase inhibitors include systemic NSAID's e.g. indomethacin, piroxicam.

The antiemetic properties of the compounds of the invention are particularly advantageous in the treatment of nausea and vomiting associated with cancer chemotherapeutic agents and radiation therapy. The compounds are therefore of use in the treatment of cancer by chemotherapeutic agents (cytotoxic or cytostatic agents such as cisplatin, doxorubicin and cyclophosphamide) as well as irradiation. Accordingly the invention also provides a product containing a cancer chemotherapeutic agent and a compound of the invention as a combined preparation for simultaneous, separate or sequential use in cancer therapy.

Preferably the pharmaceutical composition is in unit dosage form, eg as tablets or capsules. In such form, the composition is sub-divided in unit dose containing appropriate quantities of the active ingredient; the unit dosage forms can be packaged composition, for example packeted powders, vials, ampoules, prefilled syringes or sachets containing liquids. The unit dosage form can be, for example, a capsule or tablet itself, or it can be the appropriate number of any such compositions in package form.

The quantity of the active ingredient in unit dose of composition may be varied or adjusted from 0.5 mg or less to 750 mg or more, according to the particular need and the activity of the active ingredient.

The invention also includes the compounds in the absence of the carrier where the compounds are in unit dosage form.

The following Examples illustrate the invention.

EXAMPLE 1

endo-N-[[(8-Methyl-8-azabicyclo[3.2.1]octan-3-yl)amino]carbonyl]-2-cyclopropylmethoxy)benzamide

2-(Cyclopropylmethoxy)benzamide above: (17.4 g), oxalyl chloride (12.1 ml) and 1,2-dichloroethane (190

ml) were heated together at 80°C for 19 hours. The cooled solution was concentrated under reduced pressure to an oil which was re-evaporated with toluene (2 x 150 ml) to remove residual oxalyl chloride. The residual crude 2-(cyclopropylmethoxy)benzoyl isocyanate was dissolved in dry THF (230 ml).

This solution (220 ml) was added by cannula under argon to an ice-cold solution of endo-3-aminotropane (11.5 g) in dry THF (660 ml). The mixture was allowed to warm to room temperature overnight under argon and was then quenched with methanol (100 ml), stirred 3 hours, and evaporated to an oil under reduced pressure.

This oil was partitioned between 6N hydrochloric acid (200 ml) and ether (3 x 100 ml). The acid phase was made basic to pH 10 with sodium hydroxide and ice cooling, and the precipitated base was extracted into chloroform (5 x 100 ml). The extracts were dried ($Na_2SO_4$) and evaporated, leaving an oil which crystallised spontaneously (25.5 g, 88%).

The base was converted to its 1:1 maleic acid salt in propan-2-ol and crystallised twice from this solvent with charcoal treatment, giving the title salt as its 1:1 maleic acid salt (24.91 g), m.p. 162-164°C.
Found: C, 60.91; H, 6.66; N, 8.83. $C_{20}H_{27}N_3O_3.C_4H_4O_4$ requires C, 60.88; H, 6.60; N, 8.87%.

EXAMPLE 2

(endo)-N-[[(8-Methyl-8-azabicyclo[3.2.1]octan-3-yl)amino]carbonyl]-2-cyclopropylmethoxy)benzamide

(a) (endo)-N-(8-methyl-8-azabicyclo[3.2.1]octan-3-yl)-2,2,2-trichloroacetamide hydrochloride.

endo-3-Amino-8-methyl-8-azabicyclo[3.2.1]octane (7.0 g, 50 mmol) in chloroform (25 ml) was treated with hexachloroacetone (15.885 g, 60 mmol) in chloroform (35 ml). The mixture was heated to reflux for $5\frac{1}{2}$ h. Further hexachloroacetone (3.5 g, 13.2 mmol) was added and the mixture was heated again for 3 h. On cooling, several crops of the crude title compound salt were obtained by concentration of the reaction mixture. The residual oil was then diluted to 50 ml with propan-2-ol and treated with excess ethereal HCl. The precipitated crude hydrochloride was recrystallised from propan-2-ol-methanol and then from ethylacetate-methanol giving the title compound as almost colourless crystals (7.42 g) subliming and decomposing without melting above 220°.

(b) (endo)-N-[[(8-methyl-8-azabicyclo[3.2.1]octan-3-yl)amino]carbonyl]-2-(cyclopropyl)methoxybenzamide

endo-N-(8-methyl-8-azabicyclo[3.2.1]octan-3-yl)-2,2,2-trichloroacetamide base (2.745 g, 9.6 mmol) in DMSO (10 ml) was added under argon to a mixture of powdered sodium hydroxide (1.4 g, 35 mmol) and 2-(cyclopropylmethoxy)benzamide (1.91 g, 10 mmol). The mixture was then heated at 80°C for 0.5 h. The cooled mixture was poured into water (50 ml) and acidified with conc. hydrochloric acid to pH 1. The mixture was extracted with ether (4 x 25 ml) and the acid phase was then made basic to pH 10 with 2N sodium hydroxide. The precipitated oil was extracted into chloroform (4 x 25 ml). The combined extracts were washed with a mixture of saturated brine (20 ml) and 2N sodium hydroxide solution (5 ml), dried ($Na_2SO_4$), and evaporated under reduced pressure. The residual oil crystallised on standing and was recrystallised from acetonitrile giving the title compound as fawn crystals (1.535 g), m.p. 153-6°C.

## Claims

**Claims for the following Contracting States : DE, FR, IT, NL, SE, LI, CH, BE, AT, LU, DK**

**1.** (Endo)-N-[[(8-methyl-8-azabicyclo[3.2.1]octan-3-yl)amino]carbonyl]-2-(cyclopropylmethoxy)benzamide or a pharmaceutically acceptable acid addition salt thereof.

**2.** A process for preparing a compound claimed in claim 1 which comprises

(a) reacting 2-cyclopropylmethoxybenzoylisocyanate with (endo)-8-methyl-8-azabicyclo[3.2.1]octan-3-amine, or

(b) reacting 2-cyclopropylmethoxybenzoylurea with (endo)-8-methyl-8-azabicyclo[3.2.1]octan-3-amine, or

(c) reacting 2-(cyclopropylmethoxy)benzamide with (endo)-8-methyl-8-azabicyclo[3.2.1]octan-3-isocyanate or

(d) acylating 1-[(endo)-8-methyl-8-azabicyclo [3.2.1]octan-3-yl]urea with 2-(cyclopropylmethoxy)benzoic acid or an acylating derivative thereof, or

(e) cyclopropylmethylating (endo)-N-[[(8-methyl-8-azabicyclo[3.2.1]octan-3-yl)amino]carbonyl]-2-hydroxybenzamide, or

(f) reacting cyclopropylmethanol with a compound of formula

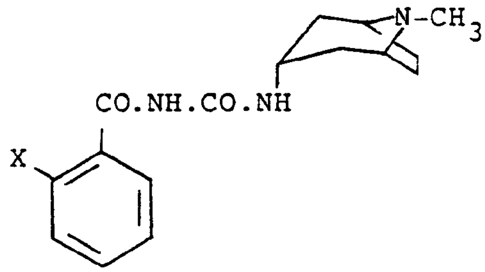

(VIII)

where X is a leaving group or
(g) methylating a compound of formula

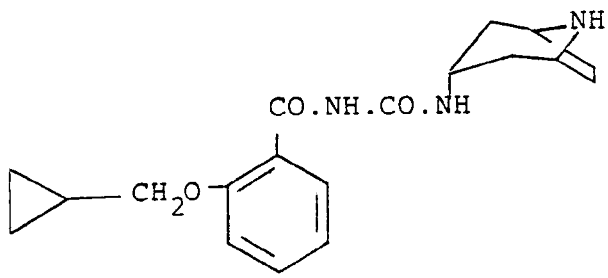

(IX)

or
(h) converting the free base (endo)-N-[[(8-methyl-8-azabicyclo[3.2.1]octan-3-yl)amino]carbonyl]-2-(cyclopropylmethoxy)benzamide, into a pharmaceutically acceptable acid addition salt thereof or converting an acid addition salt into the free base.

3. A pharmaceutical composition comprising a compound as claimed in claim 1 in association with a pharmaceutically acceptable carrier.

4. A pharmaceutical composition as claimed in claim 3 which also includes a cyclo-oxygenase inhibitor.

5. A product containing a compound as claimed in claim 1 and a cancer chemotherapeutic agent as a combined preparation for simultaneous, separate or sequential use in cancer therapy.

6. A compound as claimed in claim 1 for use as a pharmaceutical.

7. A compound as claimed in claim 1 for use as an anxiolytic agent.

8. A compound of formula

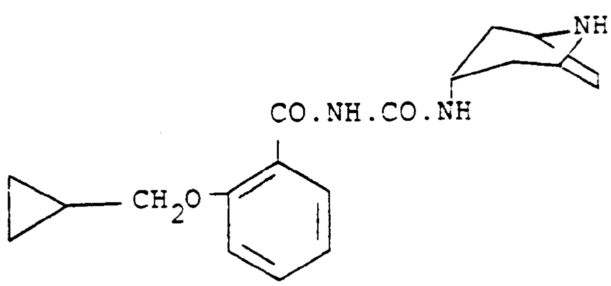

(IX)

**Claims for the following Contracting States : ES, GR**

1. A process for preparing (endo)-N-[[(8-methyl-8-azabicyclo[3.2.1]octan-3-yl)amino]carbonyl]-2-(cyclopropylmethoxy)benzamide or a pharmaceutically acceptable acid addition salt thereof which comprises

(a) reacting 2-cyclopropylmethoxybenzoylisocyanate with (endo)-8-methyl-8-azabicyclo[3.2.1]octan-3-amine, or

(b) reacting 2-cyclopropylmethoxybenzoylurea with (endo)-8-methyl-8-azabicyclo[3.2.1]octan-3-amine, or

(c) reacting 2-(cyclopropylmethoxy)benzamide with (endo)-8-methyl-8-azabicyclo[3.2.1]octan-3-isocyanate or

(d) acylating 1-[(endo)-8-methyl-8-azabicyclo [3.2.1]octan-3-yl]urea with 2-(cyclopropylmethoxy)benzoic acid or an acylating derivative thereof, or

(e) cyclopropylmethylating (endo)-N-[[(8-methyl-8-azabicyclo[3.2.1]octan-3-yl)amino]carbonyl]-2-hydroxybenzamide, or

(f) reacting cyclopropylmethanol with a compound of formula

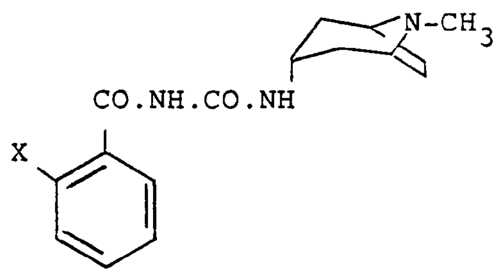

(VIII)

where X is a leaving group or

(g) methylating a compound of formula

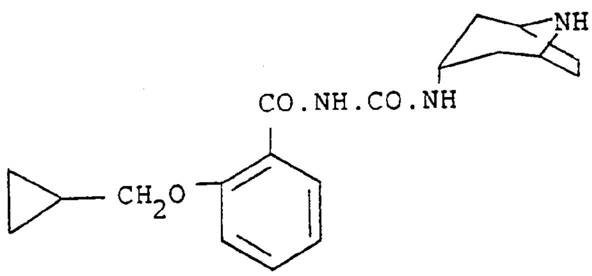

(IX)

or

(h) converting the free base, (endo)-N-[[(8-methyl-8-azabicyclo[3.2.1]octan-3-yl)amino]carbonyl]-2-(cyclopropylmethoxy)benzamide, into a pharmaceutically acceptable acid addition salt thereof or converting an acid addition salt into the free base.

**2.** A process for preparing a pharmaceutical composition which comprises bringing (endo)-N-[[(8-methyl-8-azabicyclo[3.2.1]octan-3-yl)amino]carbonyl]-2-(cyclopropylmethoxy)benzamide or a pharmaceutically acceptable acid addition salt thereof into association with a pharmaceutically acceptable acrrier.

**3.** A process as claimed in claim 2 in which a cyclo-oxygenase inhibitor is also incorporated in the composition.

**4.** A process as claimed in claim 2 or 3 in which (endo)-N-[[(8-methyl-8-azabicyclo[3.2.1]octan-3-yl)amino]carbonyl]-2-(cyclopropylmethoxy)benzamide or a pharmaceutically acceptable acid addition salt thereof is prepared by the process claimed in claim 1.

**Patentansprüche**

**Patentansprüche für folgende Vertragsstaaten : DE, FR, IT, NL, SE, LI, CH, BE, AT, LU, DK**

**1.** (Endo)-N-[[(8-Methyl-8-azabicyclo[3,2,1]octan-3-yl)-amino]-carbonyl]-2-(cyclopropylmethoxy)-benzam id oder ein pharmazeutisch annehmbares Säureadditionssalz hievon.

2. Verfahren zum Herstellen einer Verbindung nach Anspruch 1, das umfaßt:
(a) das Umsetzen von 2-Cyclopropylmethoxybenzoylisocyanat mit (endo)-8-Methyl-8-azabicyclo[3,2,1]octan-3-amin oder
(b) das Umsetzen von 2-Cyclopropylmethoxybenzoylharnstoff mit (endo)-8-Methyl-8-azabicyclo[3,2,1]octan-3-amin oder
(c) das Umsetzen von 2-(Cyclopropylmethoxy)-benzamid mit (endo)-8-Methyl-8-azabicyclo[3,2,1]octan-3-isocyanat oder
(d) das Acylieren von 1-[(endo)-8-Methyl-8-azabicyclo[3,2,1]octan-3-yl]-harnstoff mit 2-(Cyclopropylmethoxy)-benzoesäure oder einem Acylierungsderivat hievon oder
(e) das Cyclopropylmethylieren von (endo)-N-[[(8-Methyl-8-azabicyclo[3,2,1]octan-3-yl)-amino]-carbonyl]-2-hydroxybenzamid oder
(f) das Umsetzen von Cyclopropylmethanol mit einer Verbindung der Formel

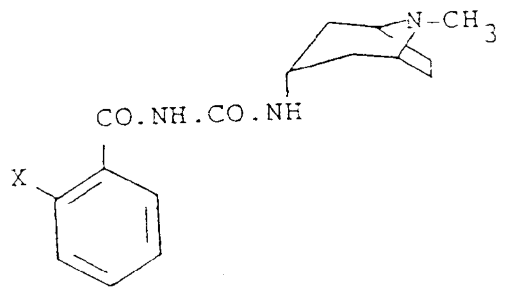

(VIII),

worin X eine Abgangsgruppe ist, oder
(g) das Methylieren einer Verbindung der Formel

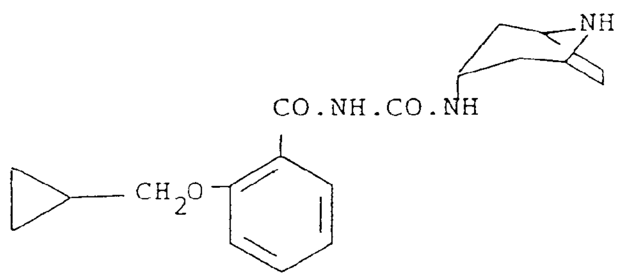

(IX)

oder
(h) das Überführen der freien Base (endo)-N-[[(8-Methyl-8-azabicyclo[3,2,1]octan-3-yl)-amino]-carbonyl]-2-(cyclopropylmethoxy)-benzamid in ein pharmazeutisch annehmbares Säureadditionssalz hievon oder das Überführen eines Säureadditionssalzes in die freie Base.

3. Pharmazeutische Zusammensetzung, die eine Verbindung nach Anspruch 1 in Assoziation mit einem pharmazeutisch annehmbaren Träger umfaßt.

4. Pharmazeutische Zusammensetzung nach Anspruch 3, die auch einen Cyclooxygenaseinhibitor aufweist.

5. Produkt enthaltend eine Verbindung nach Anspruch 1 und ein chemotherapeutisches Krebsmittel als Kombinationspräparat für die gleichzeitige, getrennte oder aufeinanderfolgende Verwendung bei der Krebstherapie.

6. Verbindung nach Anspruch 1 zur Verwendung als Pharmazeutikum.

7. Verbindung nach Anspruch 1 zur Verwendung als anxiolytisches Mittel.

8. Verbindung der Formel

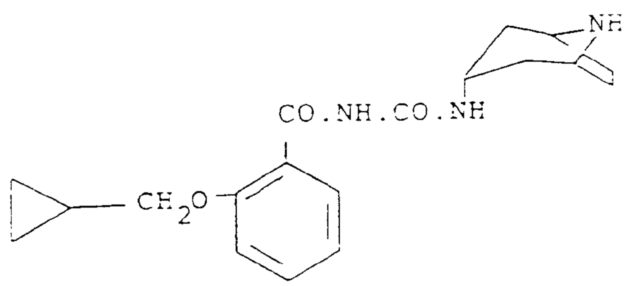

(IX).

**Patentansprüche für folgende Vertragsstaaten : ES, GR**

1. Verfahren zum Herstellen von (endo)-N-[[(8-Methyl-8-azabicyclo[3,2,1]octan-3-yl)-amino]-carbonyl]-2-(cyclopropylmethoxy)-benzamid oder eines pharmazeutisch annehmbaren Säureadditionssalzes hievon, das umfaßt:
   (a) das Umsetzen von 2-Cyclopropylmethoxybenzoylisocyanat mit (endo)-8-Methyl-8-azabicyclo[3,2,1]octan-3-amin oder
   (b) das Umsetzen von 2-Cyclopropylmethoxybenzoylharnstoff mit (endo)-8-Methyl-8-azabicyclo[3,2,1]octan-3-amin oder
   (c) das Umsetzen von 2-(Cyclopropylmethoxy)-benzamid mit (endo)-8-Methyl-8-azabicyclo[3,2,1]octan-3-isocyanat oder
   (d) das Acylieren von 1-[(endo)-8-Methyl-8-azabicyclo[3,2,1]octan-3-yl]-harnstoff mit 2-(Cyclopropylmethoxy)-benzoesäure oder einem Acylierungsderivat hievon oder
   (e) das Cyclopropylmethylieren von (endo)-N-[[(8-Methyl-8-azabicyclo[3,2,1]octan-3-yl)-amino]-carbonyl]-2-hydroxybenzamid oder
   (f) das Umsetzen von Cyclopropylmethanol mit einer Verbindung der Formel

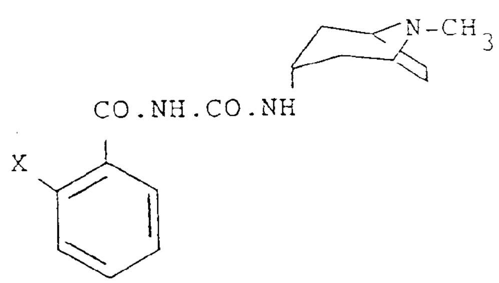

(VIII),

   worin X eine Abgangsgruppe ist, oder
   (g) das Methylieren einer Verbindung der Formel

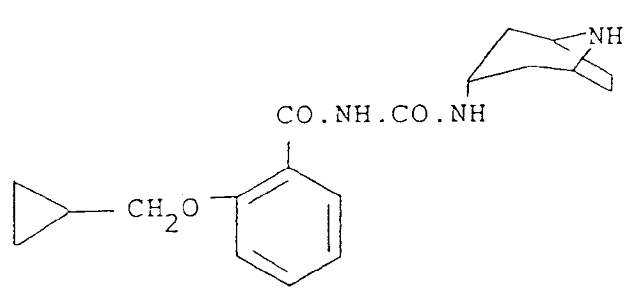

(IX)

   oder
   (h) das Überführen der freien Base (endo)-N-[[(8-Methyl-8-azabicyclo[3,2,1]octan-3-yl)-amino]-carbonyl]-2-(cyclopropylmethoxy)-benzamid in ein pharmazeutisch annehmbares Säure additionssalz hievon oder das Überführen eines Säureadditionssalzes in die freie Base.

2. Verfahren zum Herstellen einer pharmazeutischen Zusammensetzung, das das Vereinigen von (endo)-N-[[(8-Methyl-8-azabicyclo[3,2,1]octan-3-yl)-amino]-carbonyl]-2-(cyclopropylmethoxy)-benzamid oder

eines pharmazeutisch annehmbaren Säure additionssalzes hievon mit einem pharmazeutisch annehmbaren Träger umfaßt.

3. Verfahren nach Anspruch 2, in dem auch ein Cyclooxygenaseinhibitor in die Zusammensetzung eingearbeitet wird.

4. Verfahren nach Anspruch 2 oder 3, in dem (endo)-N-[[(8-Methyl-8-azabicyclo[3, 2,1]octan-3-yl)-amino]-carbonyl] -2-(cyclopropylmethoxy)-benzamid oder ein pharmazeutisch annehmbares Säureadditionssalz hievon nach dem in Anspruch 1 beanspruchten Verfahren hergestellt ist.

## Revendications

**Revendications pour les Etats contractants suivants : DE, FR, IT, NL, SE, LI, CH, BE, AT, LU, DK**

1. (endo)-N-{[(8-Méthyl-8-azabicyclo[3.2.1]octane-3-yl)amino]carbonyl}-2-(cyclopropylméthoxy)benzamide ou un sel d'addition d'acide pharmaceutiquement acceptable de celui-ci.

2. Procédé pour la préparation d'un composé suivant la revendication 1, qui comprend :
(a) la réaction de l'isocyanate de 2-cyclopropylméthoxybenzoyle avec l'(endo)-8-méthyl-8-azabicyclo[3.2.1]octane-3-amine, ou
(b) la réaction de la 2-cyclopropylméthoxybenzoylurée avec l'(endo)-8-méthyl-8-azabicyclo[3.2.1]octane-3-amine, ou
(c) la réaction du 2-(cyclopropylméthoxy)benzamide avec le 3-isocyanate d'(endo)-8-méthyl-8-azabicyclo[3.2.1]octane, ou
(d) l'acylation de la 1-[(endo)-8-méthyl-8-azabicyclo[3.2.1]octane-3-yl]urée avec l'acide 2-(cyclopropylméthoxy)benzoïque ou un dérivé acylant de celui-ci, ou
(e) la cyclopropylméthylationde l'(endo)-N-{[(8-méthyl-8-azabicyclo[3.2.1]octane-3-yl)amino]carbonyl}-2-hydroxybenzamide, ou
(f) la réaction du cyclopropylméthanol avec un composé de formule :

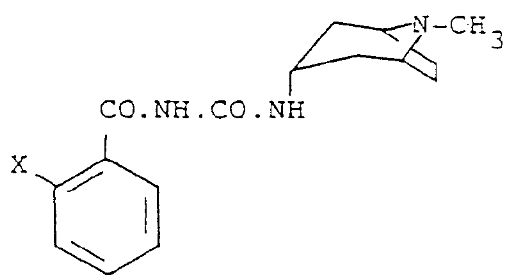

(VIII)

où X est un groupe partant, ou
(g) la méthylation d'un composé de formule :

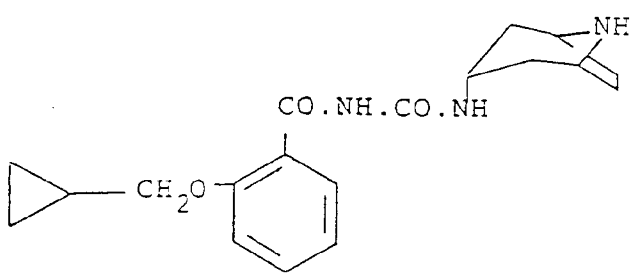

(IX)

ou,
(h) la conversion de la base libre de l'(endo)-N-{[(8-methyl-8-azabicyclo[3.2.1]octane-3-yl)amino]carbonyl}-2-(cyclopropylméthoxy)benzamide, en un sel d'addition d'acide pharmaceutiquement acceptable de celle-ci ou la conversion d'un sel d'addition d'acide en la base libre.

3. Composition pharmaceutique comprenant un composé suivant la revendication 1, en association avec un excipient pharmaceutiquement acceptable.

4. Composition pharmaceutique suivant la revendication 3, qui inclut aussi un inhibiteur de la cyclo-oxygénase.

5. Produit contenant un composé suivant la revendication 1, et un agent chimiothérapeutique anticancéreux, sous forme d'une préparation combinée pour une utilisation simultanée, séparée ou séquentielle dans la thérapie du cancer.

6. Composé la suivant la revendication 1, à utiliser comme agent pharmaceutique.

7. Composé suivant la revendication 1, à utiliser comme agent anxiolytique.

8. Composé de formule :

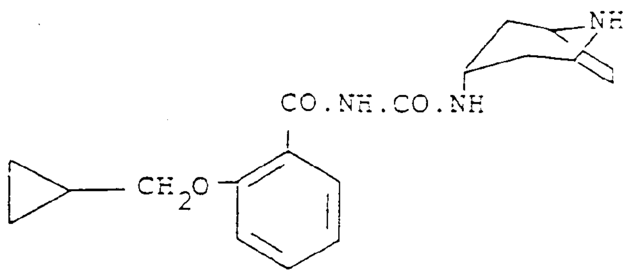

(IX)

**Revendications pour les Etats contractants suivants : ES, GR**

1. Procédé de préparation de l'(endo)-N-{[(8-méthyl-8-azabicyclo[3.2.1]octane-3-yl)amino]carbonyl}-2-(cyclopropylméthoxy)benzamide ou un sel d'addition d'acide pharmaceutiquement acceptable de celui-ci, qui comprend :
   (a) la réaction de l'isocyanate de 2-cyclopropylméthoxybenzoyle avec l' (endo)-8-méthyl-8-azabicyclo [3.2.1]octane-3-amine, ou
   (b) la réaction de la 2-cyclopropylméthoxybenzoylurée avec l'(endo)-8-méthyl-8-azabicyclo[3.2.1]octane-3-amine, ou
   (c) la réaction du 2-(cyclopropylméthoxy)benzamide avec le 3-isocyanate de l'(endo)-8-méthyl-8azabicyclo[3.2.1]octane, ou
   (d) l'acylation de la 1-[(endo)-8-méthyl-8azabicyclo[3.2.1]octane-3-yl]urée avec l'acide 2-(cyclopropylméthoxy)benzoïque ou un dérivé acylant de celui-ci, ou
   (e) la cyclopropylméthylation de l'(endo)-N-{[(8-méthyl-8-azabicyclo[3.2.1]octane-3-yl)amino]carbonyl}-2-hydroxybenzamide, ou
   (f) la réaction du cyclopropylméthanol avec un composé de formule :

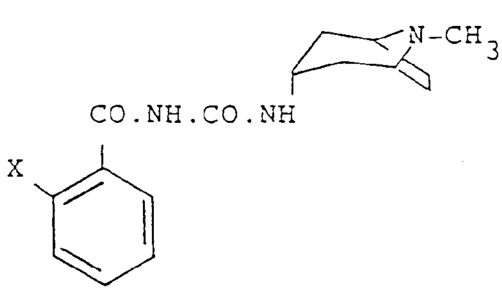

(VIII)

   où X est un groupe partant, ou
   (g) la méthylation d'un composé de formule :

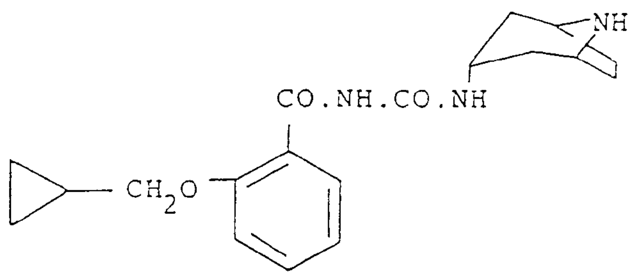

(IX)

ou

(h) la conversion de la base libre de l'(endo)-N-{[(8-méthyl-8-azabicyclo[3.2.1]octane-3-yl)amino]carbonyl}-2-(cyclopropylméthoxy)benzamide, en un sel d'addition d'acide pharmaceutiquement acceptable de celle-ci ou la conversion d'un sel d'addition d'acide en la base libre.

2. Procédé de préparation d'une composition pharmaceutique, qui comprend la mise en association de l'endo-N-{[(8-methyl-8-azabicyclo[3.2.1]octane-3-yl)amino]carbonyl}-2-(cyclopropylméthoxy)benzamide ou d'un sel d'addition d'acide pharmaceutiquement acceptable de celui-ci avec un excipient pharmaceutiquement acceptable.

3. Procédé suivant la revendication 2, dans lequel un inhibiteur de la cyclo-oxygénase est aussi incorporé dans la composition.

4. Procédé suivant la revendication 2 ou 3, dans lequel l'endo-N-{[(8-méthyl-8-azabicyclo[3.2.1]octane-3-yl)amino]carbonyl}-2-(cyclopropylméthoxy)benzamide ou un sel d'addition d'acide pharmaceutiquement acceptable de celui-ci est préparé par le procédé suivant la revendication 1.